Europäisches Patentamt

European Patent Office    (11) Publication number: **0 349 048**

.Office européen des brevets    **A2**

(19)

(12) # EUROPEAN PATENT APPLICATION

(21) Application number: 89201608.0    (51) Int. Cl.4: **A61K 35/28 , C07K 15/00 , A61K 37/00**

(22) Date of filing: 20.06.89

(30) Priority: 27.06.88 US 212399

(43) Date of publication of application:
03.01.90 Bulletin 90/01

(84) Designated Contracting States:
CH DE FR GB IT LI NL

(71) Applicant: YISSUM RESEARCH DEVELOPMENT
COMPANY OF THE HEBREW UNIVERSITY OF
JERUSALEM
46, Jabotinsky Street P.O. Box 4279
Jerusalem 91042(IL)

(72) Inventor: Bab, Itai A.
Ha'Palmach St. 36
Jerusalem(IL)
Inventor: Muhlrad, Andras
Neve Shannan St. 29a
Jerusalem(IL)
Inventor: Gazit, Dan
Ha'Palmach St. 64
Jerusalem(IL)
Inventor: Shteyer, Arie
Arazim St. 37
Mevaseret Jerusalem(IL)

(74) Representative: Hesketh, Alan, Dr.
European Patent Department Merck & Co.,
Inc. Terlings Park Eastwick Road
Harlow Essex, CM20 2QR(GB)

(54) Osteogenic growth factors derived from regenerating bone marrow.

(57) To test growth factor production, healing bone marrow-conditioned medium was prepared with tissue separated from rat tibias during the osteogenic phase and assayed for enhancement of mitogenic activity in culture of osteogenic rat osteosarcoma cells (ROS 17/2). Partial purification of healing bone marrow-conditioned medium-derived growth factor(s) consisted of gel filtration on Sephadex G-25, boiling, chromatography on heparin-Sepharose, and gel filtration on Sephadex G-75. Mitogenic activity eluted in the void volume of the Sephadex G-25 column (mol wt > 5,000). Potent activity resolved from heparin-Sepharose with PBS, and on filtration by Sephadex G-75 this activity recovered in 3 peaks with mol wt estimates of 35,000, 19,000 and less than 10,000. The partially purified factor also showed considerable stimulatory effect on DNA synthesis in osteoblastic fetal rat calvarial cells and on in vitro elongation of fetal long bone; it had only a small effect on nonosteoblastic ROS and fetal rat calvarial cells. These data indicate that healing bone marrow produces growth factor activity with a preferential effect on osteogenic cells.

# OSTEOGENIC GROWTH FACTORS DERIVED FROM REGENERATING BONE MARROW

## BACKGROUND OF THE INVENTION

It is well known that after marrow ablation there is an osteogenic phase where trabeculas of primary bone replace the blood clot and fill the marrow space. The trabeculas are then subjected to osteoblastic resorption that precedes the appearance of regenerated normal marrow. Not only is there osteogenic reaction locally in the marrow cavity, there is stimulation of bone formation in cortical osteons and enhancement of osteo- and chondrogenesis in distant skeletal sites. Observations in mandibular condyles during the osteogenic phase of postablation healing of tibial marrow suggested that the enhanced osteogenesis resulted from an increase in both the number and activity of osteoblasts. It has been proposed that a factor or factors are produced locally by the regenerating marrow that mediate the peripheral osteogenic response after their release into the blood circulation. Bab. I. et al., (1895) Calcif Tissue Int 37:551. The present invention establishes that regenerating bone marrow produces growth factor activity with a preferential effect on osteogenic cells.

## SUMMARY OF THE INVENTION

It has been shown in the present invention that regenerating bone marrow at the osteogenic phase elaborates a potent growth promoting activity to osteogenic cells. This activity, assessed by measuring the effect of HBMCM on proliferation of osteoblastic ROS cells, did not depend on the presence of serum; serum, however, enhanced this effect. The apparent increase in the recovered activity after the boiling and heparin-Sepharose steps suggests that HBMCM also contains substances inhibitory to the GFA or DNA synthesis. This idea is further supported by the inhibition of ROS cell number at the highest dilution of crude HBMCM (1:200). NMCM failed to evoke an HBMCM-like stimulation of ROS cell proliferation, suggesting that the osteogenic GFA is produced specifically during marrow healing or that the amount of such activity elaborated by normal marrow is below the resolution of the bioassay employed. Although the origin of cells producing the osteogenic GFA has not been elucidated, the microscopic examination of the osseous tissue used for preparation of HBMCM showed abundance of stromal cells, i.e. fibroblastic, angioblastic, and osteoblastic, all known to be closely associated with the osteogenic potential of bone marrow. Although the presence of hemopoietic elements could not be ruled out, their amount seems to be insufficient for the production of significant levels of bioactive factors.

Alkaline phosphatase, an enzyme found elevated in osteoblastic ROS cell cultures and a marker of differentiated osteoblast function, was also regulated by HBMCM but not NMCM. The effect of crude HBMCM on alkaline phosphatase activity was inversely related to its effect on ROS cell number. Low concentrations that suppressed cell number considerably, stimulated enzyme activity, whereas higher concentrations, at which cell number was enhanced, progressively inhibited alkaline phosphatase. A similar relationship between osteoblast proliferation and alkaline phosphatase activity is known and may suggest that replicating osteoblasts have a reduced capacity to express their differentiated cell function. However, in the absence of highly purified material, other alternatives should be considered, e.g. that the effects seen at low concentration are produced by the inhibitors of cell growth presumably present in HBMCM. Such inhibitors may affect ROS cell growth independent of the effect on alkaline phosphatase.

Both osteogenic cell populations, the ROS 17/2 and FRC populations 3-5, responded markedly to a challenge of HBMCM-derived preparations, showing a dose-response relationship. At the degree of purification achieved by the heparin-Sepharose step, the effect on DNA synthesis rates in these cell systems was apparent at doses in the nanogram range. Experiments designed herein to assess whether and to what extent HBMCM-derived preparations affect other, nonosteogenic connective tissue cells revealed that nonosteoblastic rat osteosarcoma and calvarial cells as well as mouse fibroblasts responded marginally to challenge by HBMCM derivatives. It is known that proliferation of these cells can be stimulated considerably by different hormones and growth factors; thus the present results suggest that the HBMCM derivative preferentially affects bone cells. Therefore, it appeared important to examine the effect of this factor in an intact organ culture system of long bone rudiments. Indeed, boiled HBMCM, at concentrations mitogenic to osteoblastic ROS and FRC cells, stimulated enchondral elongation of fetal radii and ulnas in a dose-response manner. The effect on total growth was somewhat higher than the stimulation of diaphyseal elongation, suggesting a differential influence on bone formation and chondrogenesis with possible involvement of both osteoblasts and epiphyseal chondrobytes. It should be pointed out that the effect of boiled HBMCM in the fetal long bone system was approximately twice that of $10^{-9}$ M insulin previously known to elicit a maximal response in the same organ culture system.

The proteinous nature of the osteogenic cell growth-promoting factor was demonstrated by the inhibition experiments with trypsin. The elution pattern from the Sephadex G-25 and G-75 columns suggests that the GFA obtained after the heparin-Sepharose step is heterogeneous with three major peaks of activity at estimated mol wts of 5,000-10,000, 19,000, and 35,000. In this mol wt range (5,000-35,000) there are several growth factors with a mitogenic effect on bone cells such as fibroblast growth factor, transforming growth factors $\alpha$ and $\beta$, $\beta_2$-Microglobulin produced in culture by fetal rat calvaria, osteoblast-derived skeletal growth factor extracted from demineralized bone matrix, and a macrophage-derived growth factor. However, the combination of features of the HBMCM-derived factor, i.e. resistance to boiling, recovery from the heparin-Sepharose bed at physiological salt concentration, and preferential effect on osteoblastic cells in the presence or absence of serum, appears to be inconsistent with the properties of most of these factors. The HBMCM-derived factor, however, shares some of its features with PDGF. Apart from its resistance to boiling, PDGF recovers from heparin-Sepharose at a relatively low NaCl concentration and acts as a promoter of osteosarcoma cell proliferation. It appears though that the present HBMCM preparations do not contain significant amount of PDGF, as determined by the antibody neutralization experiments. In addition, these preparations failed to show IL1 activity in the thymocyte proliferation assay. Transforming growth factor-$\beta$ that occurs in instances of wound healing also appears to be different from the osteogenic growth factor described herein since it inhibits proliferation of osteoblastic ROS and calvarial cells.

Instances where the osteogenic potential of bone marrow is expressed, e.g. postablation and fracture healing, marrow transplantation, in vitro marrow cell and organ cultures, and in vivo diffusion chambers cultures, have been characterized only with respect to expression of the osteogenic phenotype and its regulation by systemic hormones. To the best of our knowledge, the present invention is the first suggestion of a role for local growth factors as mediators in the sequence of events whereby bone marrow expresses its osteogenic potential. In addition to their local activity, such factors may be transferred to the blood circulation and mediate the systemic enhancement of osteogenesis that occurs during the postablatio regeneration of bone marrow.

## DETAILED DESCRIPTION OF THE INVENTION

### Materials

Reagents for assay of alkaline phosphatase activity, collagenase type I, trypsin, soybean trypsin inhibitor-agarose (STI), BGJ medium (Fitton-Jackson modification), vitamin C, bovine pancreas insulin, and human serum albumin, were purchased from Sigma Chemical Co. (St. Louis, MO). F-10 (Ham) medium (nutrient mixture), fetal calf serum (FCS), Dulbecco's PBS, and penicillin-streptomycin solution were obtained from Gibco (Chagrin Falls, OH). [methyl-$^3$H]Thymidine ([$^3$H]TdR) (5$\mu$Ci/mmol) was from Nuclear Research Center (Negev, Israel). Sehadex G-25, Sephadex G-75, and heparin-Sepharose CL-6B were purchased from Pharmacia (Uppsala, Sweden). Millipore membranes were from Schleicher & Schuell (Dassel, West Germany). Platelet-derived growth factor (PDGF) was purchased from Biomedical Technologies (Stroughton, MA) and human recombinant interleukin I-$\alpha$(IL1) from cistrone (Pine Brook, NJ). All other chemicals were of analytical grade and purchased from Merck AG (Darmstadt, West Germany). Tissue culture dishes were obtained from Nunc (Roskilde, Denmark). Rabbit polyclonal antiserum against PDGF was provided by Dr. C. H. Heldin (Uppsala University, Sweden).

### Preparation of Healing Marrow Conditioned Medium (HBMCM)

Tibial marrow was ablated from one limb of each of 400 g male rates of the Hebrew University (Sabra, Israel) strain as described previously. Bab, I. et al., (1985) Calcif Tissue Int 37:551. Briefly, a hole, 2 mm in diameter, was drilled in the shaft at a level below the proximal growth plate. Tissue was then removed from the marrow space with a polyethylene cannula inserted through the hold and attached to a high powered suction apparatus. The treated bones were dissected after 10 days and the shafts split longitudinally to expose the marrow space. Healing tissue was then removed from the endosteal aspect of the cortex, washed with copious amounts of serum-free F-10 medium supplemented with 1% (vol/vol) penicillin-streptomycin and incubated for 24 h in the same medium (tissue from one limb/1 ml medium) at 37 C in 5% $CO_2$-air. The medium was then collected and centrifuged for 30 min at 25,000 x g, and the supernatant was filtered through a 0.45-$\mu$m pore-size Millipore membrane. The preparation was designated crude HBMCM; its protein content was 3-8 mg/ml. Control medium was prepared in the same way but with normal marrow obtained from untreated rates (NMCM). To dispose of cold thymidine, components of the tissue culture medium, and other low molecular weight contaminants, the crude HBMCM was subjected to gel filtration on a Sephadex G-25 column equilibrated with 5mM ammonium-acetate. In standard experiments 6 or 35 mg protein were applied onto a PD-10 or 2.6 x 70 cm columns, respectively. Fractions were eluted with 5 mM ammonium-acetate and those containing protein in the void volume (mol wt > 5,000) were pooled lyophilized, and stored at -70 C. For further experiments samples were thawed and dissolved in PBS. The abbreviation HBMCM is used below for the preparation obtained after the Sephadex G-25 step.

Monitoring Growth Promoting Activity to Osteogenic Cells

Growth factor activity (GFA) was monitored by examining effects on DAN synthesis and cell replication in a culture of osteogenic rat osteosarcoma cells (ROS 17/2). Stock cultures of ROS-17/2 cells were maintained in F-10 medium containing 10% FCS. To study the mitogenic effect of different preparations, confluent cultures were trypsinized, and $2 \times 10^+$ cells seeded in 2 $cm^2$ culture wells (16 mm multiwell dishes) in F-10 medium and incubated at 37 C in $CO_2$-air. During the first 6 h the medium was supplemented with 2% FCS to enhance cell anchorage. This was followed by 18 h incubation in serum-free medium that contained the test preparation added as protein solution in PBS. to determine DNA synthesis rates, cultures were pulsed with [$^3$H]TdR, 1 $\mu$Ci/well, for the last 2 h of the incubation period. The pulse was terminated by washing twice with ice cold 10% (wt/vol) trichloroacetic acid and with ethanol-ether (3:1, vol/vol). After the cell layer had dried, the trichloroacetic acid-insoluble material was dissolved in 0.2 M NaOH and its total radioactivity estimated by liquid scintillation spectrometry. Data were expressed as growth factor units (GFU). Since the growth of ROS cells is serum dependent, 1 U was defined as half the effect of 10% FCS in a given experiment. Cell number was determined in sister cultures exposed to test preparations for 48 h. This was done after trypsinization using a fixed volume hemocytometer. The data were expressed as the number of cells per culture well.

Partial Purification of GFA from HBMCM

Effect of Heat

One milliliter aliquots of HBMCM containing 0.5-1.0 mg protein were left at room temperature or heated to 56° C for 30 or 60 min. Similar samples were also tested for stability of the GFA to boiling for 10 min. Since the GFA was found to be stable to the boiling procedure (Table 1) a boiling step was used for the removal of extraneous protein by denaturation and centrifugation for 45 min at 25,000 x g.

4

TABLE 1.

| Effect of heat on HBMCM mitogenic activity ([³H]TdR incorporation into DNA) in serum-free ROS 17/2 cell culture | | |
|---|---|---|
| Factor[a] | Counts per min[b] | GFR[b] |
| HBMCM (60 min, RT) | 2038 ± 304 | 0.29 ± 0.05 |
| HBMCM (30 min, 56 C) | 2792 ± 125 | 0.59 ± 0.07 |
| HBMCM (60 min, 60 C) | 3213 ± 89 | 0.82 ± 0.05 |
| HBMCM (10 min, boiling) | 5820 ± 243 | 2.82 ± 0.18 |
| Serum-free control | 1676 ± 130 | |
| Serum (10%) control | 5385 + 350 | |
| RT, Room temperature. | | |

[a]Factor concentration was 3.6 μg/ml.
[b]Mean ± SE of four replicate culture wells.

Affinity Chromatography.

A heparin-Sepharose column (0.9 x 25 cm bed volume) was prepared according to manufacturer's instructions, packed with PBS, and pumped at room temperature at a flow rate of 0.6 ml/min. Preliminary experiments using heparin-Sepharose batchwise indicated that at equilibrium with 0.15 M NaCl (PBS) the GFA was left unbound to the insoluble substrate. Thus, a 2-ml sample containing 30 mg boiling HBMCM in PBS was loaded onto the column and eluted by washing the bed with 24 ml PBS. The eluate was dialyzed for 24 h against 5 mM ammonium acetate, assessed for protein, and lyophilized.

Gel Filtration.

To further purify the HBMCM-derived factor, 0.5-6.0 mg/ml protein that recovered from the heparin-Depharose column were dissolved in 1 ml 5 mM ammonium acetate and applied onto a 1.2 x 54 cm ephadex G-75 column equilibrated with the same solution. The protein samples were eluted at room temperature using 5 mM ammonium acetate at a flow rate of .65 ml/min. Fractions, 1.3 ml, were assessed for protein and lyphilized.

Tryptic Digestion

To confirm the proteinous nature of the HBMCM-derived factor, 1-ml aliquots of HBMCM after the heparin-Sepharose step were incubated with trypsin (trypsin/HBMCM ratio 1:20 wt/wt) at 25° C. The reaction was stopped after 30 min by applying the mixture to a column of STI (0.4 x 1 cm). Controls consisted of samples treated similarly but without trypsin in the reaction mixture.

Other Cell and Organ Cultures

To study the specificity of the HBMCM-derived factor to osteogenic cells, active preparations were further tested for their mitogenic effect on osteoblastic and nonosteoblastic fetal rat calvarial cells (FRC cells), and nonosteogenic rat osteosarcoma cells (ROS 25/1). Cultures were uniformly kept at 37° C in $CO_2$-air. In all experiments test preparations were added to cultures as protein solutions in PBS.

ROS cells.

ROS 25/1 cells were cultured and tested using a protocol similar to that reported above for ROS 17/2 cells.

FRC cells.

Cells obtained from parietal bones of 21-day rat fetuses were used in primary cultures. Five cell populations were separated by sequential digestion with collagenase and trypsin according to the method described by Luben et al., (1976) Endrocrinology 99:526. Cells from populations 1-2 and 3-5 were pooled and designated nonosteoblastic and osteoblastic, respectively. The cells were seeded in 16-mm multiwell dishes, $3 \times 10^4$ cells per well, and allowed to grow for 24 h in F-10 medium supplemented with 10% FCS. The medium was then replaced by one with 1% FCS, and after 24 h the test preparation and [$^3$H]TdR, 1.5 $\mu$Ci/well, were added. Incorporation of [$^3$H]TdR into DNA and cell number were assessed as described above after an additional 24-h period.

Fetal Mouse Long Bone.

This was carried out as described by Soskolne et al., (1986) bone 7:41. Briefly, radii and ulnas were removed from 16-day fetuses and dissected free of muscle and soft tissue. They were then cultured in a chemically defined medium (BGJ, Fitton-Jackson modification) supplemented with 150 $\mu$g/ml vitamin C and 4 mg/ml human serum albumin. Phosphate concentration was adjusted to 1 mM. Total and diaphyseal lengths of individual bone rudiments were measured at the beginning of the culture period and after 48 h directly under a dissecting microscope using transmitted light. Elongation, either total or diaphyseal, was calculated as the difference between these measurements, and the results were expressed as the ratio between bones treated with growth factors and controls grown in a chemically defined medium only (T/C ratio).

Alkaline Phosphatase Activity

For this assay the medium was removed from cultures of ROS 17/2 cells, and the cells were washed with PBS, scraped into distilled water, and sonicated. Enzyme activity was assayed with p-nitrophenol phosphate as a substrate as described by Ashton et al., (1984) Calcif Tissue Int 36:83. The results were expressed as micromoles of p-nitrophenyl released per min/$10^6$ cells that were counted in sister cultures.

Protein Content

Protein was determined according to the method of Bradford, (1976) Anal. Biochem. 72:248.

Assessment of IL1 Activity

IL1 activity was estimated using a thymocyte proliferation assay as described by Barak et al., (1986) J. Biol. Response Modifies 5:362.

Assay for PDGF

Anti-PDGF antibody neutralization experiments were carried out with polyclonal antiserum prepared in rabbits against PDGF. PDGF or HBMCM-derived preparations were added to ROS 17/2 cells in the presence or absence of antibody and examined for effects on [$^3$H]TdR incorporation.

The effect of crude HBMCM and NMCM on the number and alkaline phosphatase activity of ROS 17/2 cells is established. At the highest dilution of crude HBMCM (1:200) there was a 75% decrease in cell number and more than 2-fold increase in enzyme activity. At lower dilutions, 1:100-1:10, the number of cells was approximately 2-fold higher compared to untreated cultures, and alkaline phosphatase activity showed a dose-dependent inhibition. At the dilutions tested there was only a minor effect of NMCM on proliferation and alkaline phosphatase activity of ROS 17/2 cells.

When crude HBMCM was subjected to gel filtration on Sephadex G-25 column, approximately 80% of the _aded protein recovered in fractions comprising the void volume (mol wt > 5,000). These fractions contained nearly all (94%) mitogenic activity eluted from the column.

Partial Purification of GFA From HBMCM

Effect of heat on GFA.

The effect of heat on HBMCM-derived GFA is summarized in Table 1. With the elevation of either temperature or exposure time, there was an increase in the stimulatory effect of HBMCM on [$^3$H]TdR incorporation into DNA of ROS 17.2 cells. In particular, there was a marked increase in mitogenic activity after 10 min boiling; the mitogenic effect of the supernatant obtained by boiling and centrifugation was similar to that of FCS. Half the protein could be removed by boiling and centrifugation with 8-fold increase in specific activity (Table 2). A dose-response curve of the boiled preparation in the ROS 17/2 cell assay indicated significant GFA at 0.5-5 μg/well with a decline at higher doses. Activity of the boiled preparation was also noted when 0.5 μg was added to cultures of a pool of FRC cell populations 3-5.

TABLE 2.

| Partial purification of HBMCM-derived growth factor by boiling and affinity chromatography on heparin-Sepharose | | | | |
| --- | --- | --- | --- | --- |
| Step of Purification | Protein (mg)[a] | Recovered Activity (GFU) | Specific Activity (GFU/mg) | Purification |
| Sephadex G-25 | 5 | 495 | 99 | 1 |
| Boiling | 2.5 | 2,230 | 822 | 8 |
| Heparin-sepharose | 0.5 | 30,000 | 60,000 | 606 |

[a]From 1 ml crude HBMCM.

Affinity Chromatography.

When boiled HBMCM was loaded onto a heparin-Sepharose column, 20% of the applied protein was eluted by PBS, with the remaining protein left bound. The total mitogenic activity recovered in this fraction was approximately 1,300% resulting from a marked increase in specific activity (Table 2). The enhancement of GFA after heparin-Sepharose step is also shown by the dose-response curve where the influence on ROS 17/2 cells was apparent at 50 ng/well. The peak stimulation of DNA synthesis rate was found at 0.5 μg/well with a decline thereafter. The preparation obtained using heparin-Sepharose had also a considerable mitogenic effect on osteoblastic FRC cells. When material recovered from the heparin-Sepharose column was subjected to tryptic digestion, there was more than 95% inhibition of HBMCM derived GFA (Table 3).

TABLE 3.

| Effect of tryptic digestion on stimulation of ROS 17/2 cell DNA synthesis by HBMCM chromatographed on heparin-Sepharose | | |
|---|---|---|
| Preparation added[a] | Counts per min[b] | GFU[b] |
| HBMCM[c] | 7245 ± 357 | 3.59 ± 0.28 |
| HBMCM + STI[d] | 6309 ± 683 | 2.89 ± 0.52 |
| HBMCM + trypsin + STI | 1502 ± 69 | 0.13 ± 0.05 |
| Serum-free control | 1330 ± 75 | |
| Serum (10%) control | 3998 + 68 | |

[a]In absence of serum unless otherwise specified.
[b]Mean ± SE of four replicate culture wells.
[c]Two micrograms per culture of HBMCM preparation obtained by heparin-Sepharose chromatography.
[d]Two micrograms per culture of HBMCM preparation obtained after heparin-Sepharose and STI-agarose chromatographics.

Gel Filtration on Sephadex G75.

The elution profile of GFA from the Sephadex G75 column visualized by enhancement of [³H]TdR incorporation into DNA of ROS 17/2 cells was established. Most of the protein and some mitogenic activity eluted close to the void volume of the column. Three major peaks of activity eluted in reactions 19 to 38. Based on the elution positions of mol wt markers, the mol wt estimates of the three peaks were 35,000, 19,000 and less than 10,000.

Effect HBMCM-derived Preparations In Other Cell and Organ Cultures

Nonosteoblastic ROS 25/1 cells had been obtained from the same tumor as ROS 17/2 cells but unlike the latter they do not express the osteoblastic phenotype (5). HBMCM-derived preparations, in particular those obtained after the heparin-Sepharose step, elicited some mitogenic response in the ROS 25/1 cell culture at concentrations similar to those stimulatory to ROS 17/2 cells. However, the magnitude of the ROS 25/1 cell response was considerably smaller compared to that of ROS 17/2 cells. In addition, the HBMCM derived preparations did not have an apparent effect on DNA synthesis rates of nonosteoblastic FRC cells (populations 1-2).

When boiled HBMCM was added to an organ culture of fetal radii and ulnas there was a marked dose-dependent enhancement of growth expressed by increases in both diaphyseal and total elongation. The peak effect was found at 8 μg/ml protein concentration. At this concentration the increase of diaphyseal and total lengths was approximately 200% and 250% over growth factor-free controls, respectively. The difference in the magnitude of elongation between the diaphysis and whole rudiment resulted from enhanced growth of the cartilaginous epiphyseal ends. The peak effect of boiled HBMCM was nearly twice that of a positive insulin control.

Thymocyte Proliferation Assay for IL1 Activity

Table 4 shows that unlike IL1 preparations, in medium containing PHA, neither boiled HBMCM nor the derivative obtained after the heparin-Sepharose step stimulated [³H]TdR incorporation into thymocyte DNA, suggesting that the HBMCM-derived growth factor does not resemble IL1.

TABLE 4.

| Effect of IL1 and HBMCM preparations on incorporation [³H]TdR into DNA of isolated murine thymocytes | | |
|---|---|---|
| Factor[a] | Concentration[b] | Counts per min[b] |
| Boiled HBMCM | 0.4 μg/ml | 1770 ± 13 |
| | 4.0 μg/ml | 1613 ± 11 |
| HS-HBMCM[c] | 0.7 μg/ml | 2667 ± 5 |
| | 1.1 μg/ml | 2290 ± 17 |
| IL1[d] | 5.0 U/ml | 7634 ± 35 |
| IL1[e] | 5.0 U/ml | 6532 ± 30 |
| PHA | 10.0 μg/ml | 2590 ± 20 |
| -PHA | | 400 + 46 |
| HBMCM and IL1 preparations were tested in the presence of 10.0 ml PHA. Mean ± SE of eight culture microwells. HBMCM after heparin-Sepharose step. From human monocytes. Human recombinant. | | |

PDGF Content

Addition of polyclonal anti-PDGF antibodies to ROS 17/2 cells inhibited PDGF-stimulated replication but did not reduce the mitogenic effect produced by intermediate doses of boiled HBMCM and the preparation obtained after the heparin-Sepharose step (Fig. 8). Under these conditions the presence of significant amount of PDGF in the HBMCM-derived preparations should have resulted in decreased enhancement of ROS cell proliferation when tested with the antiserum.

**Claims**

1. Osteogenic growth factors having a molecular weight of 5,000-10,000, 19,000 or 35,000.
2. The factors of Claim 1 having a molecular weight of 5,000-10,000.
3. The factors of Claim 1 having a molecular weight of 19,000.
4. The factors of Claim 1 having a molecular weight of 35,000.
5. The factors of Claim 1 that are stable after boiling at 100° C for 10 minutes.
6. The factors of Claim 5 that are recovered from a haparin-Sepharose column at physiological salt concentration.
7. The factors of Claim 6 that are recovered with 0.15M NaCl.
8. The factors of Claim 7 that are proteinous.
9. The factors of Claim 1 derived from bone marrow.
10. The factors of Claim 9 wherein the bone marrow is regenerating.